## Europäisches Patentamt

## European Patent Office

## Office européen des brevets

(19)

(11) Veröffentlichungsnummer: **0 045 414**
**B1**

(12) **EUROPÄISCHE PATENTSCHRIFT**

(45) Veröffentlichungstag der Patentschrift:
**25.01.84**

(51) Int. Cl.³: **C 07 D 231/52**

(21) Anmeldenummer: **81105597.9**

(22) Anmeldetag: **16.07.81**

(54) **Verfahren zur Herstellung von 3-Amino-1-phenylpyrazol-5-on.**

(30) Priorität: **23.07.80 DE 3027845**

(43) Veröffentlichungstag der Anmeldung:
**10.02.82 Patentblatt 82/6**

(45) Bekanntmachung des Hinweises auf die Patenterteilung:
**25.01.84 Patentblatt 84/4**

(84) Benannte Vertragsstaaten:
**CH DE FR GB LI**

(56) Entgegenhaltungen:
**DE - A - 1 900 125**
**DE - A - 1 955 920**
**FR - A - 2 044 125**
**FR - A - 2 130 941**

(73) Patentinhaber: **HOECHST AKTIENGESELLSCHAFT,**
**Postfach 80 03 20, D-6230 Frankfurt am Main 80 (DE)**

(72) Erfinder: **Tronich, Wolfgang, Dr., Adolf-Guckes-Weg 5,**
**D-6239 Eppstein/Taunus (DE)**
Erfinder: **Rieper, Wolfgang, Dr., Beethovenstrasse 56,**
**D-6000 Frankfurt am Main (DE)**
Erfinder: **Böhme, Peter, Dr., c/o Kasei Hoechst Co. Ltd.,**
**3810 Chihama, Daitoh-cho, Ogasa-gun, Shizuoka Pref.**
**(JP)**

Verfahren zur Herstellung von 3-Amino-1-phenylpyrazol-5-on

In Org. Synth., Coll. Vol. 3, 708–709 ist die Herstellung von 3-Amino-1-phenyl-pyrazol-5-on aus einem Mol Cyanessigsäureethylester, einem Mol Phenylhydrazin und zwei Mol Natriumethylat beschrieben. Hierbei wird zur heissen Lösung des Natriumethylats in Ethanol zunächst der Cyanessigester und anschliessend das Phenylhydrazin gegeben, die Mischung unter Rühren und Druck 16 Stunden auf 120 °C erhitzt, das Ethanol weitgehend im Vakuum abdestilliert, der Rückstand mit Wasser versetzt, mehrfach mit Ether extrahiert, die wässrige Phase mit Eisessig angesäuert und das ausgefällte Produkt zunächst mit Ethanol gewaschen, dann damit ausgekocht, nach Kühlen erneut mit Ethanol gewaschen und getrocknet. Man erhält ein braunes Produkt vom Schmelzpunkt 216 bis 218 °C (unter Zersetzung) in einer Ausbeute von 43 bis 47%. Es wird bemerkt, dass man meistens ein sehr stark gefärbtes Produkt erhält, wenn man an Stelle der beschriebenen Aufarbeitung das Reaktionsgemisch nach dem 16-stündigen Erhitzen direkt mit Wasser versteht und die Lösung ansäuert. Weiterhin wird hervorgehoben, dass mindestens zwei Äquivalente Natriummethylat für die Reaktion notwendig sind, dass jedoch grössere Mengen die Ausbeute nicht verbessern.

Überraschenderweise wurde nun gefunden, dass mit nur 1 bis 1,2 Mol des Alkoholats das Produkt in besserer Qualität und besserer Ausbeute erhalten wird, wenn der Cyanessigester zur vorgelegten Mischung aus Phenylhydrazin und Alkoholat in einem polaren Lösemittel zudosiert wird.

Gegenstand der Erfindung ist deshalb ein Verfahren zur Herstellung von 3-Amino-1-phenyl-pyrazol-5-on durch Erhitzen von Phenylhydrazin und einem niederen Cyanessigsäurealkylester in einem polaren Lösemittel mit einem niederen Alkalimetallalkoholat als Kondensationsmittel, das dadurch gekennzeichnet ist, dass das Phenylhydrazin und 1 bis 1,2 Mol des Alkoholats vorgelegt werden, der Cyanessigester zudosiert wird und die Reaktion bei 105 bis 140 °C durchgeführt wird, wobei aus dem Ester und gegebenenfalls aus dem Alkoholat freigesetzte, bei der Reaktionstemperatur flüchtige Alkohole abdestilliert werden.

Hierbei wird das Verfahrensprodukt nach einer Reaktionszeit von nur wenigen Stunden in guter Ausbeute und in Form von fast farblosen Kristallen erhalten. Die Reinheit des Produktes ist so hoch, dass es für alle Anforderungen ohne weitere Reinigung eingesetzt werden kann.

Im folgenden werden bevorzugte Ausführungsformen der Erfindung näher beschrieben:

Es wurde gefunden, dass die Ausbeute erheblich gesteigert werden kann, wenn das Phenylhydrazin im Überschuss eingesetzt wird. So führt bereits ein Überschuss von 10 Mol–% Phenylhydrazin zu einer Ausbeute von über 50% der Theorie, ein Überschuss von etwa 200 Mol–% in die Grössenordnung von 70% der Theorie. Aus wirtschaftlichen Erwägungen wird ein Überschuss von etwa 10 bis etwa 80 Mol–% besonders bevorzugt.

Als polares Lösemittel kommen alle organischen Substanzen in Betracht, die ein entsprechendes Lösevermögen und hinreichende Stabilität gegen das Alkalialkoholat bei den Reaktionstemperaturen aufweisen. Bevorzugt sind Alkohole oder Ether mit einem Siedepunkt über 105 °C, beispielsweise Alkanole mit mindestens 4 Kohlenstoffatomen und Glykole sowie deren Ether, beispielsweise Diethylendiglykol, Diethylenglykolmono- und diether. Besonders bevorzugt werden n-Butanol und insbesondere iso-Butanol.

Als Ester der Cyanessigsäure sind die niederen Alkylester bevorzugt, insbesondere der Methyl- und Ethylester. Werden Ester von Alkoholen mit einem Siedepunkt über 105 °C in Kombination mit geeigneten Alkoholaten eingesetzt, so erübrigt sich die Abtrennung des bei der Reaktion freigesetzten Alkohols.

Als Alkalialkoholat wird bevorzugt ein niederes Natriumalkanolat verwendet, insbesondere Natriummmethylat. Das Alkoholat kann in fester Form eingesetzt werden, vorteilhaft jedoch als Lösung, zweckmässig im zugrundeliegenden Alkohol. Besonders bevorzugt wird Natriummethylat als etwa 30%ige Lösung in Methanol. Überraschenderweise hat es sich gezeigt, dass bei der erfindungsgemässen Reaktionsführung nicht nur etwa stöchiometrische Mengen an Alkoholat ausreichen, sondern dass die nach dem Stand der Technik für erforderlich gehaltene Menge von 2 Mol sogar zu geringeren Ausbeuten führt.

Auch bei Temperaturen, die deutlich unter den 120 °C des bekannten Verfahrens liegen, wird bei der erfindungsgemässen Durchführung der Reaktion nach wenigen Stunden eine hohe Ausbeute erzielt. Temperaturen über 140 °C sind nicht vorteilhaft. Druck ist nicht erforderlich.

In einer besonders bevorzugten Ausführungsform wird Isobutanol vorgelegt, etwa 1,1 Mol Natriummethylat als etwa 30%ige Lösung in Methanol zugegeben und das Methanol bei Temperaturen bis etwa 115 °C weitgehend abdestilliert. Man gibt überschüssiges Phenylhydrazin zu und tropft bei einer Temperatur von etwa 105 bis etwa 115 °C Cyanessigsäuremethylester zu. Das freigesetzte Methanol wird kontinuierlich abdestilliert; die Umsetzung ist beendet, wenn kein Methanol mehr übergeht. Dies ist nach etwa 2 bis 3 Stunden der Fall.

Zur Aufarbeitung wird das Reaktionsgemisch zweckmässig mit Wasser gemischt, vorteilhaft durch Eingiessen in Wasser, und das Produkt mit Säure gefällt. Vorzugsweise wird hierbei ein pH-Wert von 6 nicht unterschritten. Von Vorteil ist es, wenn vor oder bei der Fällung ein Reduktionsmittel zugesetzt wird, beispielsweise dem Wasser ein Alkalimetallsulfit, -disulfit oder -dithionit, insbesondere Natriumdithionit.

Zur Fällung kann jede genügend starke organische oder anorganische Säure verwendet werden, bevorzugt sind Mineralsäuren wie Salzsäure. Bevorzugt wird die Fällung bei Erreichen eines pH-Wertes von 6,5 abgebrochen und das Produkt durch Filtration isoliert.

Bei Verwendung eines wenig mit Wasser mischbaren Lösemittels, beispielsweise Isobutanol, hat es sich als zweckmässig erwiesen, einen wasserlöslichen Alkohol zuzusetzen, vorzugsweise der bei der Umsetzung freigesetzte und abdestillierte Alkohol, und erst dann das Reaktionsgemisch mit Wasser zu mischen. Auf diese Weise wird ein besonders reines Produkt erhalten.

In den folgenden Beispielen beziehen sich Teile und Prozentangaben auf das Gewicht, sofern nichts anderes angegeben ist. Alle Umsetzungen wurden unter Stickstoff durchgeführt.

Beispiele

In einem Rührkolben werden 300 Teile Isobutanol und 250 Teile einer etwa 30%igen Natriummethylatlösung in Methanol (1,1 Mol) vorgelegt. Man destilliert das Methanol über eine Füllkörperkolonne bei einer Sumpftemperatur von 115 °C weitgehend ab und lässt dann die unten angegebene Menge Phenylhydrazin zulaufen. Anschliessend tropft man innerhalb von etwa 2 Stunden 125 Teile Cyanessigsäuremethylester (1 Mol) zu, und destilliert laufend isobutanolhaltiges Methanol ab. Wenn bei einer maximalen Sumpftemperatur von 115 °C kein Destillat mehr übergeht, ist die Reaktion beendet.

Man lässt nun das Destillat zum Ansatz zurücklaufen, rührt die Mischung in etwa 380 Teile Wasser ein und stellt mit Salzsäure einen pH-Wert von 6,5 ein. Das ausgefallene Produkt wird abgesaugt, mit Wasser gewaschen und im Vakuum getrocknet. Man erhält 3-Amino-1-phenyl-pyrazol-5-on in Form leicht cremefarbener Kristalle in dünnschichtchromatographisch reiner Form. Der Schmelzpunkt liegt bei 218–219 °C.

| Beispiel | Mol Phenylhydrazin | Ausbeute (% der Theorie) |
|---|---|---|
| 1 | 1,0 | 47,0 |
| 2 | 1,1 | 52,0 |
| 3 | 1,2 | 54,0 |
| 4 | 1,8 | 62,4 |
| 5 | 3 | 68,1 |

Vergleichsbeispiele

Beispiel 6

Führt man die Umsetzung entsprechend Beispiel 2 durch, jedoch mit 2 Mol Natriummethylat, so beträgt die Ausbeute nur 44% der Theorie.

Beispiel 7

Führt man die Umsetzung entsprechend Beispiel 2 durch, jedoch im geschlossenen Gefäss, also ohne Abdestillieren des Methanols, so erhält man nach 3 Stunden Erhitzen auf 125 °C eine Ausbeute von 41% der Theorie. Das Produkt ist braun gefärbt.

Beispiel 8

Wiederholt man Beispiel 2, jedoch mit 300 Teilen Methanol anstelle des eingesetzten Isobutanols und führt die Reaktion 3 Stunden bei 125 °C im Autoklav durch, so erhält man eine Ausbeute von nur 30% der Theorie.

Beispiel 9

Führt man die Umsetzung entsprechend Beispiel 2 durch, gibt jedoch alle Komponenten gemeinsam zu Beginn der Reaktion zu und destilliert das gesamte Methanol innerhalb von 6 Stunden ab, so erhält man nur eine Ausbeute von 34% der Theorie.

**Patentansprüche:**

1. Verfahren zur Herstellung von 3-Amino-1-phenyl-pyrazol-5-on durch Erhitzen von Phenylhydrazin und einem niederen Cyanessigsäurealkylester in einem polaren Lösemittel mit einem niederen Alkalimetallalkoholat als Kondensationsmittel, dadurch gekennzeichnet, dass das Phenylhydrazin im Überschuss, bezogen auf den Cyanessigester, und 1 bis 1,2 Mol des Alkoholats vorgelegt werden, der Cyanessigester zudosiert wird und die Reaktion bei 105 bis 140 °C durchgeführt wird, wobei aus dem Ester und gegebenenfalls aus dem Alkoholat freigesetzte bei der Reaktionstemperatur flüchtige Alkohole abdestilliert werden.

2. Verfahren nach Anspruch 1, dadurch gekennzeichnet, dass das Phenylhydrazin in einem Überschuss bis zu 200 Mol.-% eingesetzt wird.

3. Verfahren nach Anspruch 1 und 2, dadurch gekennzeichnet, dass ein Überschuss von 10 bis 80 Mol.-% an Phenylhydrazin eingesetzt wird.

4. Verfahren nach Anspruch 1 bis 3, dadurch gekennzeichnet, dass das polare Lösemittel ein Alkohol oder Ether mit einem Siedepunkt über 105 °C ist.

5. Verfahren nach Anspruch 1 bis 4, dadurch gekennzeichnet, dass das Lösemittel iso-Butanol ist.

6. Verfahren nach Anspruch 1 bis 5, dadurch gekennzeichnet, dass Cyanessigsäuremethylester in Gegenwart von Natriummethylat umgesetzt wird.

7. Verfahren nach Anspruch 1 bis 6, dadurch gekennzeichnet, dass man methanolische Natriummethylatlösung in iso-Butanol vorlegt, weitgehend methanolfrei destilliert, das Phenylhydrazin zusetzt, Cyanessigsäuremethylester zudosiert und das freiwerdende Methanol abdestilliert.

8. Verfahren nach Anspruch 1 bis 7, dadurch gekennzeichnet, dass das Reaktionsgemisch mit Wasser gemischt und das Produkt mit Säure bei einem pH-Wert nicht unter 6 gefällt wird.

9. Verfahren nach Anspruch 8, dadurch gekennzeichnet, dass vor oder bei der Fällung ein Alkalimetallsulfit-, disulfit oder -dithionit als Reduktionsmittel zugesetzt wird.

10. Verfahren nach Anspruch 8 und 9, dadurch gekennzeichnet, dass bei Verwendung von n- oder iso-Butanol als wenig mit Wasser mischbarem Lösemittel der abdestillierte flüchtige Alkohol dem Reaktionsgemisch zugesetzt wird, bevor dieses mit Wasser gemischt wird.

**Revendications**

1. Procédé de préparation de l'amino-3 phényl-1 pyrazolone-5 par chauffage de la phénylhydrazine et d'un cyanacétate d'alkyle inférieur dans un solvant polaire avec un alcoolate inférieur de métal alcalin comme agent de condensation, procédé en ce qu'on commence par placer dans le récipient réactionnel la phénylhydrazine, en une quantité représentant un excès par rapport à l'ester cyanacétique, et de 1 à 1,2 mole de l'alcoolate, on introduit progressivement l'ester cyanacétique et on effectue la réaction à une température de 105 à 140 °C, les alcools volatils à la température réactionnelle qui sont libérés à partir de l'ester et, éventuellement, à partir de l'alcoolate étant chassés par distillation.

2. Procédé selon la revendication 1, caractérisé en ce qu'on utilise la phénylhydrazine en un excès allant jusqu'à 200% en moles.

3. Procédé selon l'une des revendications 1 et 2, caractérisé en ce qu'on utilise un excès de phénylhydrazine représentant de 10 à 80% en moles.

4. Procédé selon l'une quelconque des revendications 1 à 3, caractérisé en ce que le solvant polaire est un alcool ou un éther ayant un point d'ébullition supérieur à 105 °C.

5. Procédé selon l'une quelconque des revendications 1 à 4, caractérisé en ce que le solvant est l'isobutanol.

6. Procédé selon l'une quelconque des revendications 1 à 5, caractérisé en ce qu'on fait réagir le cyanacétate de méthyle en présence de méthylate de sodium.

7. Procédé selon l'une quelconque des revendications 1 à 6, caractérisé en ce qu'on commence par introduire dans le récipient une solution méthanolique de méthylate de sodium dans de l'isobutanol, on chasse par distillation la plus grande partie du méthanol, on ajoute la phénylhydrazine, on fait arriver progressivement le cyanacétate de méthyle et on chasse par distillation le méthanol libéré.

8. Procédé selon l'une quelconque des revendications 1 à 7, caractérisé en ce qu'on ajoute de l'eau au mélange réactionnel et on fait précipiter le produit par un acide à un pH non inférieur à 6.

9. Procédé selon la revendication 8, caractérisé en ce qu'avant ou pendant la précipitation, on ajoute un sulfite, un disulfite ou un dithionite de métal alcalin comme réducteur.

10. Procédé selon l'une des revendications 8 et 9, caractérisé en ce que dans le cas où l'on utilise du n-butanol ou de l'isobutanol comme solvant peu miscible à l'eau, on ajoute au mélange réactionnel l'alcool volatil séparé par distillation avant d'ajouter de l'eau à ce mélange.

**Claims:**

1. Process for the manufacture of 3-amino-1-phenyl-pyrazol-5-one by heating phenylhydrazine and a lower cyanoacetic acid alkyl ester in a polar solvent with a lower alcoholate of an alkali metal as the condensation agent, which comprises initially taking the phenylhydrazine in an excess referred to the cyano acetic ester and 1 to 1.2 moles of the alcoholate, metering in the cyanoacetic ester and carrying out the reaction at 105 to 140 °C, distilling off alcohols being volatile at the reaction temperature which have been liberated from the ester and optionally from the alcoholate.

2. Process as claimed in claim 1, wherein the phenylhydrazine is employed in an excess of up to 200 mole %.

3. Process as claimed in claims 1 and 2, wherein an excess of 10 to 80 mole % of phenylhydrazine is employed.

4. Process as claimed in claims 1 to 3, wherein the polar solvent is an alcohol or ether having a boiling point above 105 °C.

5. Process as claimed in claims 1 to 4, wherein the solvent is isobutanol.

6. Process as claimed in claims 1 to 5, wherein cyanoacetic acid methyl ester is reacted in the presence of sodium methylate.

7. Process as claimed in claims 1 to 6, wherein methanolic sodium methylate solution in isobutanol is initially taken and is distilled so that it is largely free from methanol, the phenylhydrazine is added, cyanoacetic acid methyl ester is metered in and the methanol liberated is distilled off.

8. Process as claimed in claims 1 to 7, wherein the reaction mixture is mixed with water and the product is precipitated with acid at a pH value not below 6.

9. Process as claimed in claim 8, wherein an alkalimetal sulfite, -disulfite or -dithionite as a reducing agent is added before or during the precipitation.

10. Process as claimed in claims 8 and 9, wherein, if n- or iso-butanol as a solvent which is slightly miscible with water is used, the volatile alcohol which has been removed by distillation is added to the reaction mixture before the latter is mixed with water.